# EUROPÄISCHE PATENTSCHRIFT

(11) **EP 1 718 601 B1**
(45) Veröffentlichungstag und Bekanntmachung des Hinweises auf die Patenterteilung: **25.03.2009**
(21) Anmeldenummer: 04821681.6
(22) Anmeldetag: 02.12.2004
(51) Int. Cl.: C07C 225/22

(54) **(3,5-DIAMINOPHENYL)(2,4-DIHYDROXY-PHENYL)METHANON UND DESSEN SÄUREADDUKTE, VERFAHREN ZU DEREN HERSTELLUNG UND DIE VERWENDUNG DIESER VERBINDUNGEN ZUR FÄRBUNG VON FASERN**
(3,5-DIAMINOPHENYL)(2,4-DIHYDROXYPHENYL)METHANONE AND ITS ACID ADDUCTS, METHOD FOR THE PRODUCTION OF THE LATTER AND USE OF SAID COMPOUNDS FOR COLOURING FIBRES
(3,5-DIAMINOPHENYL)(2,4-DIHYDROXY-PHENYL)METHANONE ET SES PRODUITS D'ADDITION ACIDE, LEUR PROCEDE DE PREPARATION ET L'UTILISATION DE CES COMPOSES POUR TEINDRE DES FIBRES

(30) Priorität: 24.02.2004 DE 102004008918
(43) Veröffentlichungstag der Anmeldung: 08.11.2006
(73) Patentinhaber: Wella Aktiengesellschaft, 64274 Darmstadt (DE)
(72) Erfinder: GÖTTEL, Otto, CH-1723 Marly (CH); HAYOZ, André, CH-1724 Senèdes (CH); BRAUN, Hans-Jürgen, CH-3182 Ueberstorf (CH)
(74) Vertreter: Bockhorst, Matthias
(86) Internationale Anmeldenummer: PCT/EP2004/013704
(87) Internationale Veröffentlichungsnummer: WO 2005/080315

(56) Entgegenhaltungen:
- DE-A1- 10 128 472
- DE-A1- 10 217 270

## Beschreibung

Gegenstand der vorliegenden Erfindung sind das (3,5-Diaminophenyl)-(2,4-dihydroxy-phenyl)methanon sowie dessen Säureaddukte, ein Verfahren zur Herstellung dieser Verbindungen sowie die Verwendung dieser Verbindungen zur Färbung von Fasern.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels. Von besonderem Interesse sind nach wie vor Haarfärbemittel zur Abdeckung des Naturtonbereichs. Bisher konnte der wichtige Bereich der Naturtöne, vor allem der dunkleren Nuancen, nur durch komplexe Mischungen von mehreren verschiedenen Entwicklersubstanzen und Kupplersubstanzen abgedeckt werden. Normalerweise wurden dazu bisher Kombinationen von Paraphenylendiaminen mit Resorcinen, m-Aminophenolen und m-Phenylendiaminen verwendet, wobei heute aus verschiedenen Gründen kaum noch die Grundkörper, sondern überwiegend deren Derivate eingesetzt werden. Insbesondere wegen der komplexen Zusammensetzungen der mittleren bis dunkleren Naturtöne bestand der Wunsch nach Komponenten, mit denen Brauntöne erzielt werden können, ohne dass hierzu komplexe Zusammensetzungen einer Vielzahl von einzelnen Farbkomponenten benötigt werden, wobei eine 1 :1-Zusammensetzung einer einzigen Entwicklersubstanz und einer einzigen Kupplersubstanz besonders interessant sind.

Kupplerkomponenten sind aus DE-10128472 und DE-10217270 bekannt.

Es bestand daher die Aufgabe, eine Kupplerkomponente zu finden, die in der Lage ist, zusammen mit geeigneten Entwicklern Brauntöne zu erzeugen und Färbungen mit einer hohen Stabilität gegenüber den Alltagsbedingungen, wie zum Beispiel häufigem Waschen, ergibt. Weiterhin sollen die beim vorzeitigen Verschießen einzelner Farben in komplexen Zusammensetzungen häufig auftretenden unerwünschten Farbverschiebungen vermieden werden.

Überraschenderweise wurde nunmehr gefunden, dass der Kuppler der Formel (I) und dessen Salze die gestellte Aufgabe in hervorragender Weise lösen.

Gegenstand der vorliegenden Erfindung sind daher das (3,5-Diaminophenyl)(2,4-dihydroxy-phenyl)methanon sowie dessen Säureaddukte gemäß Formel (I), mit 0 ≤ n ≥ 2, und HX gleich einer anorganischen oder organischen Säure.

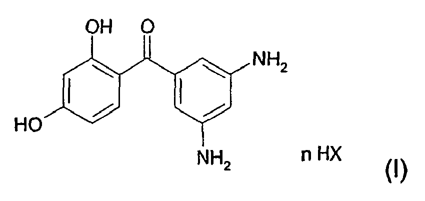

Als anorganische oder organische Säure können Salzsäure, Schwefelsäure, Phosphorsäure, Zitronensäure oder Weinsäure genannt werden, wobei Salzsäure und Schwefelsäure besonders bevorzugt sind.

Die Herstellung der Verbindungen der Formel (I) erfolgt gemäß Schema I aus 3,5-Dinitro-benzoylchlorid und Resorcin unter Friedel-Crafts-Bedingungen. Das Zwischenprodukt wird katalytisch zu m Endprodukt der Formel (I) hydriert, das entweder als freie Base oder als Säureaddukt isoliert werden kann.

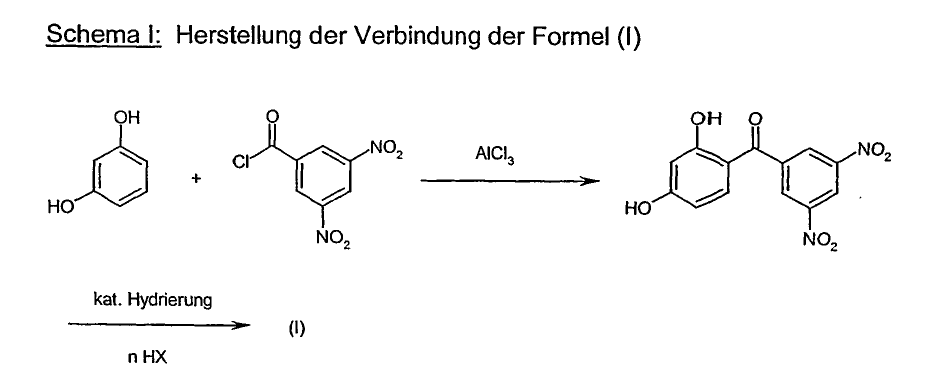

Ein weiterer Gegenstand der Erfindung ist die Verwendung der Verbindungen der Formel (I) allein oder in Kombination mit bestimmten Entwicklersubstanzen und Kupplersubstanzen zur oxidativen Färbung von synthetischen oder natürlichen Fasermaterialien. Unter den natürlichen Fasermaterialien eignen sich beispielsweise keratinischen Fasern wie zum Beispiel Wolle oder Haare und insbesondere menschliche Haare.

Als Farbentwickler, die sich in hervorragender Weise zur Kombination in einer oxidativen Formulierung eignen, kommen Verbindungen des Paraphenylendiamin-, Paraaminophenol- sowie 4,5-Diaminopyrazol-Typs in Frage, die selbstverständlich auch als Säureaddukte vorliegen können. Dazu zählen insbesondere 1,4-Diamino-benzol (p-Phenylendiamin), 1,4-Diamino-2-methyl-benzol (p-Toluylen-diamin), 1,4-Diamino-2,6-dimethyl-benzol, 1,4-Diamino-2,5-dimethyl-benzol, 1,4-Diamino-2,3-dimethyl-benzol, 2-Chlor-1,4-diamino-benzol, 4-Phenylamino-anilin, 4-Dimethylamino-anilin, 4-Diethylamino-anilin, 4-[Di(2-hydroxyethyl)-amino]-anilin, 1,4-Diamino-2-methoxymethyl-benzol, 1,4-Diamino-2-aminmethyl-benzol, 1,4-Diamino-2-phenyl-benzol, 4-[(2-Methoxyethyl)-amino]-anilin, 4-[(3-Hydroxypropyl)-amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methyl-phenyl)methyl]-1 H-pyrazol, 1-[(4-Chlorphenyl)-methyl]-4,5-diamino-1H-pyrazol, 4,5-Diamino-1-methyl-1 H-pyrazol, 4,5-Diamino-1-pentyl-1H-pyrazol, 4,5-Diamino-3-methyl-1-phenyl-1H-pyrazol, 4,5-Diamino-1-(2-hydroxyethyl)-3-methyl-1H-pyrazol, 1,2-Bis-(4,5-diamino-1H-pyrazol-1-yl)-ethan, 1,4-Bis-(4,5-diamino-pyrazol-1-yl-methyl)-benzol, 4,5-Diamino-1-(2-methylphenyl)-1 H-pyrazol, 4,5-Diamino-1-(3-methylphenyl)-1 H-pyrazol, 4,5-Diamino-1-(4-methylphenyl)-1H-pyrazol, 4,5-Diamino-1-(2,4-dimethylphenyl)-1H-pyrazol, 4,5-Diamino-1-(2,5-dimethylphenyl)-1H-pyrazol, 4,5-Diamino-1-(2-ethylphenyl)-1H-pyrazol, 4,5-Diamino-1-(4-isopropylphenyl)-1H-pyrazol, 4,5-Diamino-1-(4-methoxyphenyl)-1H-pyrazol,1-(4-Amino-phenyl)-4,5-diamino-1H-pyrazol, 1-(4-Chlorphenyl)-4,5-diamino-1H-pyrazol, 4,5-Diamino-1-(2-pyridinyl)-1H-pyrazol,2-Amino-phenol,2-Amino-6-methyl-phenol und 2-Amino-5-methyl-phenol, oder deren Salze.

Als mögliche Kuppler, die zur Erzeugung bestimmter Nuancen in Kombination mit den erfindungsgemäßen Verbindungen der Formel (I) eingesetzt werden können, können die folgenden Verbindungen genannt werden: N-(3-Dimethylamino-phenyl)-harnstoff, 2,6-Diamino-pyridin, 2-Amino-4-[(2-hydroxyethyl)amino]-anisol,2,4-Diamino-1-fluor-5-methylbenzol, 2,4-Diamino-1-methoxy-5-methyl-benzol,2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol,2,4-Diamino-1-(2-hydroxyethoxy)-benzol, 2,4-Diamino-1-(3-hydroxypropoxy)-benzol,1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol,5-Methyl-2-(1-methylethyl)-phenol,3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol, 7-Hydroxy-indol und 2,3-Indolindion, sowie deren Salze.

Die vorstehend beschriebenen Verbindungen der Formel (I) werden zur Färbung in einer geeigneten Farbträgermasse appliziert.

Ein weiterer Gegenstand der vorliegenden Erfindung ist daher ein gebrauchsfertiges Mittel zur oxidativen Färbung von Keratinfasern, insbesondere Haaren, das durch Vermischen einer Farbträgermasse mit einem Oxidationsmittel unmittelbar vor der Anwendung hergestellt wird und dadurch gekennzeichnet ist, dass die Farbträgermasse mindestens eine Verbindung der Formel (I) enthält.

Die Verbindungen der Formel (I) sind in dem erfindungsgemäßen Färbemittel (bezogen auf die Farbträgermasse) vorzugsweise in einer Menge von 0,01 bis 10 Gewichtsprozent, insbesondere 0,1 bis 6 Gewichtsprozent, enthalten.

Das erfindungsgemäße Färbemittel kann neben den Verbindungen der Formel (I) sowie den vorgenannten Entwicklersubstanzen und Kupplersubstanzen weiterhin auch direktziehende Farbstoffe aus der Gruppe der anionischen, kationischen oder neutralen Farbstoffe enthalten. Zu den bevorzugten anionischen Farbstoffen zählen beispielsweise 6-Hydroxy-5-[(4-sulfophenyl)azo]-2-naphthalin-sulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; 1; Food Yellow No. 1), 2-(Indan-1,3-dion-2-yl)chinolin-x,x-sulfonsäure (Gemisch aus Mono- und Disulfonsäure) (C147005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylamino-benzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäure-natriumsalz (CI15510; Acid Orange No. 7), 4-[(2,4-Dihydroxy-3-[(2,4-dimethylphenyl)azo]-phenyl)azo]-benzolsulfonsäure-natriumsalz (CI20170; Acid Orange No. 24), 4-Hydroxy-3-[(4-sulfonaphth-1-yl)azo]-1-naphthalin-sulfonsäuredinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalindisulfonsäure-trinatriumsalz (CI16185; Acid Red No. 27), 8-Amino-1-hydroxy-2-(phenylazo)-3,6-naphthalin-disulfonsäure-dinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanaminiumhydroxid, inneres Salz, Natriumsalz (CI45100; Acid Red No. 52), 8-[(4-(Phenylazo)phenyl)azo]-7-naphthol-1,3-disulfonsäure-dinatriumsalz (CI27290; Acid Red No. 73), 2',4',5',7'-Tetrabrom-3',6'-dihydroxyspiro-[isobenzofuran-1 (3H),9'-[9H]xanthen]-3-on-dinatriumsalz (CI45380; Acid Red No. 87), 2',4',5',7'-Tetrabrom-4,5,6,7-tetrachlor-3',6'-dihydroxyspiro-[isobenzofuran-1 (3H),9'[9H]xanthen]-3-on-dinatriumsalz (CI45410; Acid Red No. 92), 3',6'-Dihydroxy-4',5'-diiodospiro[isobenzofuran-1(3H),9'(9H)-xanthen)-3-on-dinatriumsalz (CI45425; Acid Red No. 95), (2-Sulfophenyl)di[4-(ethyl((4-sulfophenyl)methyl)amino)phenyl]-carbeniumdinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)-phenyl](2,4-disulfophenyl)carbenium-inneres salz, Natriumsalz (2:1) (CI42045; Food Blue No. 3; Acid Blue No. 1), Bis[4-(diethylamino)phenyl]-(5-hydroxy-2,4-disulfophenyl)carbenium-inneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1 H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis{ 3-nitro-4-[(4-phenylamino)-3-sulfo-phenylamino]-phenyl }-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäuredinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäure-natriumsalz Chrom-Komplex (Acid Red No. 195).

Zum besseren Farbausgleich und zur Erzeugung von speziellen Nuancen sind die folgenden nichtionischen Farbstoffe besonders geeignet: 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)-amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)-amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin und 2-((4-(Acetylamino)phenyl)-azo)-4-methylphenol (CI11855; Disperse Yellow No. 3).

Aus der Gruppe der direktziehenden Farbstoffe besonders zu erwähnen sind weiterhin 2-Amino-4,6-dinitrophenol, 2-Ethylamino-4,6-dinitrophenol, 2-[(2-Hydroxyethyl)amino]-4,6-dinitrophenol und Farbstoffe der allgemeinen Formel (II), 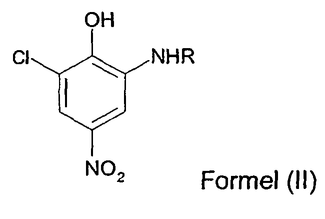 worin R Wasserstoff, Methyl, Ethyl oder Hydroxyethyl bedeutet.

Die Gesamtmenge an Oxidationsfarbstoffvorstufen beträgt (bezogen auf die Farbträgermasse) 0,01 bis 10 Gewichtsprozent, insbesondere 0,2 bis 6 Gewichtsprozent.

Die Gesamtmenge an direktziehenden Farbstoffen in der Farbträgermasse liegt zwischen 0,1 und 10 Gewichtsprozent, wobei eine Gesamtmenge 0,1 bis 5 Gewichtsprozent bevorzugt ist.

Darüber hinaus können in der Farbträgermasse noch Antioxidantien (beispielsweise, Natriumsulfit und/oder Ascorbinsäure), Parfümöle, Komplexbildner, Netzmittel, Emulgatoren, Penetrationsmittel, Puffersysteme, Konservierungsstoffe, Verdicker, Pflegestoffe und andere kosmetische Zusätze enthalten sein.

Die Zubereitungsform für die Farbträgermasse sowie für das gebrauchsfertige Oxidationshaarfärbemittel kann beispielsweise eine Lösung, insbesondere eine wässrige oder wässrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze für Lösungen, Cremes, Emulsionen oder Gele sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionischen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalko hole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet. Bezogen auf die Farbträgermasse sind dies zum Beispiel Netzmittel und Emulgatoren in Konzentrationen von etwa 0,5 bis 30 Gewichtsprozent, Verdicker in einer Menge von etwa 0,1 bis 25 Gewichtsprozent und die Pflegestoffe in einer Konzentration von etwa 0,1 bis 5 Gewichtsprozent.

Das gebrauchsfertige erfindungsgemäße Haarfärbemittel wird durch Mischen der Farbträgermasse mit einem flüssigen Oxidationsmittel unmittelbar vor der Anwendung hergestellt.

Als Oxidatiönsmittel kommen hauptsächlich Wasserstoffperoxid oder dessen Additionsverbindungen an Harnstoff, Melamin oder Natriumbromat in Form einer 1- bis 12-prozentigen, vorzugsweise 6-prozentigen, wäßrigen Lösung in Betracht, wobei Wasserstoffperoxid besonders bevorzugt ist.

Die Farbträgermasse und das Oxidationsmittel werden hierbei im Gewichtsverhältnis von 5 zu 1 bis 1 zu 3 miteinander vermischt, wobei ein Gewichtsverhältnis von 1 bis 1 bis 1 zu 2 besonders bevorzugt ist.

Der pH-Wert des gebrauchsfertigen erfindungsgemäßen Haarfärbemittels stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird. Der pH-Wert des gebrauchsfertigen Haarfärbemittels kann zwischen 3 und 11, bevorzugt zwischen 6 und 10,5 liegen.

Für die Einstellung des jeweiligen pH-Wertes der Farbträgermasse und des Oxidationsmittels können je nach dem gewünschten pH-Wert organische oder anorganische Säuren, wie zum Beispiel Phosphorsäure, Ascorbinsäure und Milchsäure, oder Alkalien wie Monoethanolamin, Triethanolamin, 2-Amino-2-methyl-1-propanol, Ammoniak, Natronlauge, Kalilauge oder Tris(hydroxymethyl)amino-methan, verwendet werden.

Nach der Vermischung der vorstehend beschriebenen Farbträgermasse mit dem Oxidationsmittel wird eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar aufgetragen.

Man läßt das erfindungsgemäße Haarfärbemittel bei etwa 10 bis 45 Minuten und 15 bis 50 Grad Celsius, vorzugsweise 30 Minuten bei 40 Grad Celsius auf das Haar einwirken, und spült dann das Haar mit Wasser aus. Gegebenenfalls wird im Anschluß an diese Spülung mit einem Shampoo gewaschen und eventuell mit einer verdünnten schwachen organischen Säure, wie zum Beispiel Zitronensäure oder Weinsäure nachgespült. Anschließend wird das Haar getrocknet.

Die nachfolgenden Beispiele sollen den Gegenstand näher erläutern, ohne ihn jedoch auf diese Beispiele zu beschränken.

### Beispiele

### Beispiel 1: Herstellung von (3,5-Diaminophenyl)(2,4-dihydroxyphenyl)methanon

### Stufe 1: (2,4-Dihydroxyphenyl)(3,5-dinitrophenyl)methanon

17,6 g (160 mmol) Resorcin werden in 80 ml Sulfolan (Tetrahydrothiophen-1,1-dioxid) gelöst. Anschließend gibt man 21,6 g (160 mmol) Aluminiumchlorid zu. Bei 60°C werden dann innerhalb von 20 Minuten portionsweise 34 g (148 mmol) 3,5-Dinitrobenzoylchlorid eingetragen, wobei sich Salzsäuregas entwickelt. Nach beendeter Zugabe wird noch 4 Stunden lang auf 90 °C erwärmt. Anschließend wird die Reaktionsmischung abgekühlt und vorsichtig mit 320 ml einer 5-prozentigen Salzsäure versetzt. Der entstehende Nierderschlag wird durch Dekantieren und Verwerfen der überstehenden Lösung abgetrennt, mit 400 ml Ethanol versetzt und durch Rühren bei Raumtemperatur zur Kristallisation gebracht. Nach dem Absaugen und Trocknen im Vakuum erhält man 40 g eines gelblichen Produkts.
¹H-NMR (DMSO-*d₆*): δ = 11,32 ppm (s, 1 H); 10,78 ppm (s, 1H); 9,98 ppm (t, ⁴J_{HH} = 2,1 Hz, 1H); 8,73 ppm (d, ⁴J_{HH} = 2,1 Hz, 2H); 7,44 ppm (dd, ³J_{HH} = 8,1 Hz, ⁴J_{HH} = 0,9 Hz, 1H); 6,43 ppm (d, ⁴J_{HH} = 2,1 Hz, 1H); 6,40 ppm (s, 1H)

| Elementaranalyse: | C₁₃H₈N₂O₇; M = 304,21 | | | |
|---|---|---|---|---|
| | | %C | %H | %N |
| | ber.: | 51,33 | 2,65 | 9,21 |
| | gef.: | 50,95 | 2,68 | 8,87 |

### Stufe 2: (3,5-Diaminophenyl)(2,4-dihydroxyphenyl)methanondihydrochlorid

25,0 g (82 mmol) werden in 300 ml Ethanol an 2,5 g Pd/C 10% bei 9 bar Wasserstoffdruck hydriert. Nach 6 Stunden wird der Katalysator abfiltriert, das Filtrat mit 100 ml Salzsäure 32% versetzt und anschließend am Rotationsverdampfer aufkonzentriert, wobei das Produkt allmählich auskristallisiert. Es entsteht ein Kristallbrei, der zur Vervollständigung der Kristallisation noch 1 Stunde im Eisbad gekühlt wird. Man saugt ab, wäscht den Rückstand mit Essigester und trocknet bei 40 °C im Vakuum. Es werden 18,3 g (70% der Theorie) eines beigefarbenen Produktes erhalten.
¹H-NMR (DMSO-*d₆*): δ = 8,60 (s breit, 8H); 7,38 (d, ³J_{HH} = 9,3 Hz, 1H); 7,01 (Signalüberlagerung, 3H); 6,42 ppm (Signalüberlagerung, 2H).
¹³C-NMR (DMSO-*d₆*): δ = 196,0 (CO); 165, 0 (C-OH); 163,7 (C-OH); 140,1 (2 C-NH2); 136,7 (C); 134,7 (CH); 117,9 (2 CH); 116,5 (CH); 112,5 (C); 108,3 (CH); 102,8 ppm (CH).

| Elementaranalyse: | C₁₃H₁₂N₂O₃ 2 HCl; M = 317,17; 1,3% Kristallwasser | | | | |
|---|---|---|---|---|---|
| | | %C | %H | %N | %Cl |
| | ber.: | 48,84 | 5,16 | 8,76 | 21,07 |
| | gef.: | 48,74 | 5,08 | 8,58 | 20,91 |

### Stufe 3: (3,5-Diaminophenyl)(2,4-dihydroxyphenyl)methanon

1 g (3,1 mmol) (3,5-Diaminophenyl)(2,4-dihydroxyphenyl)methanondihydrochlorid werden in 30 ml Wasser gelöst und durch Zugabe einer gesättigten Natriumhydrogencarbonatlösung neutralisiert. Es bildet sich dabei ein gelblicher Niederschlag, der abgesaugt wird. Nachwaschen mit wenig kaltem Wasser und Trocknen im Vakuum bei 40 °C ergeben 0,6 g (78% der Theorie) gelbliches Pulver.
FAB-MS: 245 [M+1]⁺, 100%
¹H-NMR (DMSO-*d₆*): δ = 12,52 (s, 1H); 10,66 (s, 1H); 7,52 (d, ³J_{HH} = 8,7 Hz, 1H); 6,35 (Signalüberlagerung, 2H); 6,02 (m zentriert, 3H); 5,05 ppm (s verbreitert, 5H).

### Beispiel 2: Haarfärbemittel

### Farbträgermasse

| | |
|---|---|
| 10,0 g | Ethanol |
| 10,0 g | Natriumlaurylethersulfat, 28%ige wäßrige Lösung |
| 10,0 g | Ammoniak, 25%ige wässrige Lösung |
| 0,3 g | Ascorbinsäure |
| 0,4 g | (3,5-Diaminophenyl)(2,4-dihydroxyphenyl)methanondihydrochlorid |
| X g | Entwicklersubstanz entsprechend Tabelle 1 |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf Haare aufgetragen. Nach einer Einwirkzeit von 30 Minuten bei 40 °C wird das Haar mit einem Shampoo gewaschen, mit Wasser gespült und getrocknet. Man erhält die in Tabelle 1 genannten Nuancen.

**Tabelle 1**

| **Beispiel Nr.** | **Menge** | **Entwicklersubstanz** | **Farbton** |
|---|---|---|---|
| **2a** | 0,28 g | 2,5-Diaminotoluol-sulfat | hellbraun |
| **2b** | 0,32 g | 1,4-Diamino-2-(2-hydroxyethyl)-benzol-sulfat (1:1) | hellbraun |
| **2c** | 0,16 g | 4-Amino-3-methyl-phenol | aschblond |
| **2d** | 0,30 g | 4,5-Diamino-1-(2-hydroxyethyl)-1H-pyrazol-sulfat (1:1) | rotviolett |
| **2e** | 0,37 g | 4-(Di(2-hydroxyethyl)amino)-anilin-sulfat (1:1) | mittelbraun |
| **2f** | 0,14 g | 2,5-Diaminotoluol-sulfat | weinrot |
| | 0,15g | 4,5-Diamino-1-(2-hydroxyethyl)-1H-pyrazol-sulfat (1:1) | |

### Beispiel 3: Haarfärbecreme

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| 4,50 g | Ammoniak, 25%ige wässrige Lösung |
| 0,55 g | 2,5-Diaminotoluol-sulfat |
| 0,79 g | (3,5-Diaminophenyl)(2,4-dihydroxyphenyl)methanon-dihydrochlorid |
| ad 100,00 g | Wasser, entmineralisiert |

Der pH-Wert der Creme liegt bei 10,2.

Unmittelbar vor der Anwendung werden 100 Gramm der vorstehenden Farbträgermasse mit 100 Gramm einer 6%igen Wasserstoffperoxidemulsion vermischt und das erhaltene gebrauchsfertige Oxidationshaarfärbemittel in der erforderlichen Menge auf Haare aufgetragen. Nach einer Einwirkzeit von 30 Minuten bei 40 °C wird das Haar mit einem Shampoo gewaschen, mit Wasser gespült und getrocknet.

Die erhaltenen Farbnuancen auf verschiedenen Haarqualitäten sind in Tabelle 2 zusammengefaßt.

**Tabelle 2**

| **Haartyp** | **Nuance** |
|---|---|
| gebleichtes Haar | mittel- bis dunkelbraun |
| zu 50% ergrautes Humanhaar | mittelbraun, sehr gute Grauabdeckung |
| Humanhaar der Tontiefe 7/0 | mittelbraun |

Alle Prozentangaben in der vorliegenden Anmeldung stellen, sofern nicht anders angegeben, Gewichtsprozente dar.

## Patentansprüche

1. (3,5-Diamino-phenyl)(2,4-dihydroxy-phenyl)methanon sowie dessen Säureaddukte der Formel (I), mit 0 ≤ n ≥ 2, und HX gleich einer anorganischen oder organischen Säure.

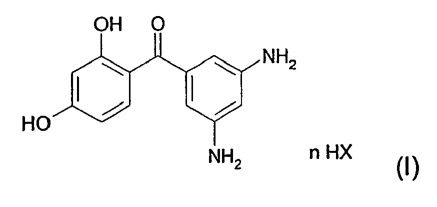

2. Verbindung nach Anspruch 1, **dadurch gekennzeichnet dass** die Säure HX ausgewählt ist aus Salzsäure, Schwefelsäure, Phosphorsäure, Zitronensäure und Weinsäure.

3. Gebrauchsfertiges Mittel zur oxidativen Färbung von Keratinfasern, das durch Vermischen einer Farbträgermasse mit einem Oxidationsmittel unmittelbar vor der Anwendung hergestellt wird und **dadurch gekennzeichnet ist, dass** die Farbträgermasse mindestens eine Verbindung der Formel (I) nach Anspruch 1 oder 2 enthält.

4. Mittel nach Anspruch 3, **dadurch gekennzeichnet, dass** die Verbindung der Formel (I) in einer Menge von 0,01 bis 10 Gewichtsprozent (bezogen auf die Farbträgermasse) enthalten ist.

5. Mittel nach Anspruch 3 oder 4, **dadurch gekennzeichnet, dass** es mindestens eine Entwicklersubstanz enthält.

6. Mittel nach einem der Ansprüche 3 bis 5, **dadurch gekennzeichnet**, das es zusätzlich weitere Entwicklersubstanzen und /oder Kupplersubstanzen und/oder direktziehende Farbstoffe enthält.

7. Mittel nach Anspruch 5 oder 6, **dadurch gekennzeichnet, dass** die Gesamtmenge an Entwicklersubstanzen und Kupplersubstanzen (bezogen auf die Farbträgermasse) 0,01 bis 10 Gewichtsprozent beträgt.

8. Mittel nach Anspruch 6 oder 7, **dadurch gekennzeichnet, dass** die Gesamtmenge an direktziehenden Farbstoffen (bezogen auf die Farbträgermasse) 0,1 bis 10 Gewichtsprozent beträgt.

9. Mittel nach einem der Ansprüche 3 bis 8, **dadurch gekennzeichnet, dass** es ein Haarfärbemittel ist.

10. Verwendung der Verbindungen der Formel (I) nach Anspruch 1 oder 2 zur oxidativen Färbung von synthetischen oder natürlichen Fasermaterialien.

11. Verfahren zur Herstellung der Verbindungen der Formel (I) nach Anspruch 1 oder 2, bei dem zunächst 3,5-Dinitro-benzoylchlorid und Resorcin unter Friedel-Crafts-Bedingungen miteinander umgesetzt werden und sodann das erhaltene Produkt katalytisch zum Endprodukt der Formel (I) hydriert wird, welches abschließend entweder als freie Base oder als Säureaddukt isoliert wird.

## Claims

1. (3,5-Diaminophenyl)(2,4-dihydroxyphenyl)methanone and its acid adducts of the formula (I), where 0 ≤ n ≥ 2, and HX is an inorganic or organic acid.

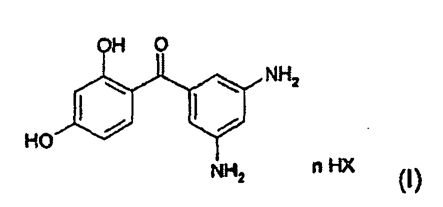

2. Compound according to Claim 1, **characterized in that** the acid HX is selected from hydrochloric acid, sulphuric acid, phosphoric acid, citric acid and tartaric acid.

3. Ready-to-use composition for the oxidative colouring of keratin fibres, which is prepared by mixing a colour carrier mass with an oxidizing agent directly prior to use and is **characterized in that** the colour carrier mass comprises at least one compound of the formula (I) according to Claim 1 or 2.

4. Composition according to Claim 3, **characterized in that** the compound of the formula (I) is present in an amount of from 0.01 to 10 per cent by weight (based on the colour carrier mass).

5. Composition according to Claim 3 or 4, **characterized in that** it comprises at least one developer substance.

6. Composition according to one of Claims 3 to 5, **characterized in that** it additionally comprises further developer substances and/or coupler substances and/or direct dyes.

7. Composition according to Claim 5 or 6, **characterized in that** the total amount of developer substances and coupler substances (based on the colour carrier mass) is 0.01 to 10 per cent by weight.

8. Composition according to Claim 6 or 7, **characterized in that** the total amount of direct dyes (based on the colour carrier mass) is 0.1 to 10 per cent by weight.

9. Composition according to one of Claims 3 to 8, **characterized in that** it is a hair colorant.

10. Use of the compounds of the formula (I) according to Claim 1 or 2 for the oxidative colouring of synthetic or natural fibre materials.

11. Method for producing the compounds of the formula (I) according to Claim 1 or 2, in which firstly 3,5-dinitrobenzoyl chloride and resorcinol are reacted together under Friedel-Crafts conditions and then the resulting product is catalytically hydrogenated to give the end product of the formula (I), which is finally isolated either as free base or as acid adduct.

## Revendications

1. (3,5-diamino-phényl)(2,4-dihydroxy-phényl)-méthanone ainsi que ses produits d'addition avec des acides de formule (I), où 0 ≤ n ≥ 2, et HX représente un acide organique ou inorganique

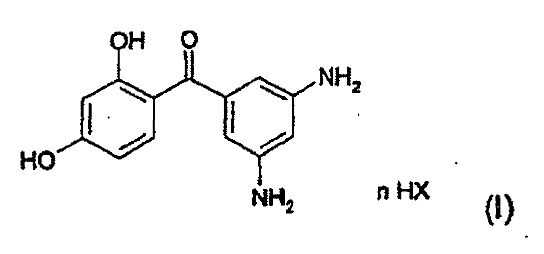

2. Composé selon la revendication 1, **caractérisé en ce que** l'acide HX est choisi parmi l'acide chlorhydrique, l'acide sulfurique, l'acide phosphorique, l'acide citrique et l'acide d-tartrique.

3. Composition prête à l'emploi pour la teinture par oxydation de fibres de kératine, qui est préparée immédiatement avant l'emploi, par mélange d'une matière colorante avec un oxydant, et est **caractérisée en ce que** la matière colorante contient au moins un composé de formule (I) selon la revendication 1 ou 2.

4. Composition selon la revendication 3, **caractérisée en ce que** le composé de formule (I) est contenu en une quantité de 0,01 à 10 % en poids (par rapport à la matière colorante)..

5. Composition selon la revendication 3 ou 4, **caractérisée en ce qu'**elle contient au moins un développeur.

6. Composition selon l'une quelconque des revendications 3 à 5, **caractérisée en ce qu'**elle contient en outre d'autres développeurs et/ou des coupleurs et/ou des colorants directs.

7. Composition selon la revendication 5 ou 6, **caractérisée en ce que** la quantité totale de développeurs et de coupleurs (par rapport à la matière colorante) va de 0,01 à 10 % en poids.

8. Composition selon la revendication 6 ou 7, **caractérisée en ce que** la quantité totale de colorants directs (par rapport à la matière colorante) va de 0,1 à 10 % en poids.

9. Composition selon l'une quelconque des revendications 3 à 8, **caractérisée en ce qu'**elle est une composition de teinture pour cheveux.

10. Utilisation des composés de formule (I) selon la revendication 1 ou 2, pour la teinture par oxydation de matières fibreuses naturelles ou synthétiques.

11. Procédé pour la préparation des composés de formule (I) selon la revendication 1 ou 2, dans lequel on fait d'abord réagir l'un avec l'autre du chlorure de 3,5-dinitro-benzoyle et du résorcinol, dans des conditions de Friedel-Crafts et ensuite le produit obtenu est hydrogéné par voie catalytique en le produit final de formule (I), qui est enfin isolé soit sous forme de base libre, soit sous forme de produit d'addition avec un acide.
